# EUROPÄISCHE PATENTANMELDUNG

(11) **EP 1 974 767 A1**
(43) Veröffentlichungstag der Anmeldung: **01.10.2008**
(21) Anmeldenummer: 07023412.5
(22) Anmeldetag: 04.12.2007
(51) Int. Cl.: A61N 1/375, H01R 24/04

(54) **Federkontakt und Kontaktbuchse für einen Elektrodenleitungsstecker**

(30) Priorität: 24.03.2007 DE 102007014219
(71) Anmelder: BIOTRONIK CRM Patent AG, 6341 Baar (CH)
(72) Erfinder: Rebentisch, Ronald, 10967 Berlin (DE)
(74) Vertreter: Lindner-Vogt, Karin L.

(57) **Zusammenfassung**

Die Erfindung betrifft eine Kontaktbuchse (16) zum Anschluss einer Elektrodenleitung an ein implantierbares medizinisches Gerät, mit einem Buchsengehäuse, welches einen längsgestreckten Aufnahmeraum (18) mit einer Längsachse zum Aufnehmen eines Steckers entlang der Längsachse aufweist, und wenigstens einem in dem Buchsengehäuse angeordneten elektrisch leitenden Kontaktelement (22), welches über wenigstens einen Federkontakt (38) verfügt, der beim Einführen eines Steckers federnd elastisch nach außen ausweicht, wodurch eine nach innen wirkende Feder-Rückstellkraft aufgebaut wird, wobei das Kontaktelement (22) wenigstens eine flache Scheibe (32) mit einer zentralen Öffnung umfasst, welche über zwei ebene Stirnflächen, sowie eine äußere Umfangsfläche und eine, die zentrale Öffnung begrenzende innere Fläche verfügt, wobei der Federkontakt (38) Teil der flachen Scheibe (32) ist, als Vorsprung der inneren Fläche in die zentrale Öffnung (36) hineinragt und über eine Kontaktierfläche verfügt, die dazu ausgebildet ist, bei in die Kontaktbuchse eingeführtem Stecker mit einer Gegenkontaktfläche des Steckers eine elektrische Verbindung herzustellen.

## Beschreibung

Die Erfindung betrifft eine Kontaktbuchse zum Anschließen einer Elektrodenleitung an ein implantierbares medizinisches Gerät, insbesondere an einen implantierbaren Herzschrittmacher oder einen implantierbaren Kardioverter/Defibrillator.

Derartige Kontaktbuchsen besitzen ein Buchsengehäuse mit einer Steckeraufnahme mit einem längsgestreckten Aufnahmeraum zur Aufnahme eines passenden Steckers.

Bei Herzschrittmachern oder Defibrillatoren dient die Kontaktbuchse dazu, einen Stecker am proximalen Ende einer Elektrodenleitung einerseits dicht mit dem Gehäuse des Herzschrittmachers oder Defibrillators zu verbinden und gleichzeitig eine sichere und zuverlässige elektrische Verbindung herzustellen. Über den Stecker am proximalen Ende der Elektrodenleitung werden üblicherweise im Bereich des distalen Endes der Elektrodenleitung vorgesehene Elektroden elektrisch mit elektrischen und/oder elektronischen Komponenten im Inneren des Gehäuses des Herzschrittmachers oder Defibrillators verbunden. Zu diesen elektrischen und/oder elektronischen Komponenten zählen beispielsweise Stimulationsimpulsgeneratoren oder Eingangsverstärker zur Verstärkung von im Herzen aufgenommenen elektrischen Potentialen.

Die Kontaktbuchse ist üblicherweise in einem Anschlussstück angeordnet, welches selbst aus isolierendem Kunststoff besteht und dicht mit einem übrigen Gehäuse eines Herzschrittmachers oder Defibrillators verbunden ist. Das übrige Gehäuse besteht üblicherweise aus Metall und schließt die elektrischen und/oder elektronischen Komponenten ein. Die elektrische Verbindung zwischen den Komponenten im Inneren des Gehäuses des Herzschrittmachers und dem Anschlussstück sowie der im Anschlussstück angeordneten Kontaktbuchse erfolgt über elektrische Durchführungen.

In der Kontaktbuchse selbst sind elektrisch leitende Kontaktelemente angeordnet, mit denen entsprechende Gegenkontakte des Steckers an der Elektrodenleitung kontaktiert werden, das heißt, elektrisch leitend verbunden werden können.

Um eine Austauschbarkeit von Elektroden bzw. Herzschrittmachern und Defibrillatoren verschiedener Hersteller zu ermöglichen, sind die Anschlussmaße des Steckers an der Elektrodenleitung sowie die entsprechenden Maße der Kontaktbuchse im Anschlussstück des Herzschrittmachers oder Defibrillators standardisiert. Der derzeit am weitesten verbreitete Standard wird üblicherweise mit IS-1 bezeichnet. Ein neuer, aktueller Standard ist unter der Bezeichnung IS-4 bekannt.

Der Erfindung liegt die Aufgabe zugrunde, eine Kontaktbuchse zu schaffen, die eine zuverlässige Herstellung eines Anschlussstücks, insbesondere für einen Herzschrittmacher oder Defibrillator erlaubt und die darüber hinaus eine zuverlässige elektrische Verbindung mit den Gegenkontakten am Stecker einer Elektrodenleitung gewährleistet oder die eine zuverlässige Abdichtung der Kontaktbuchse mit eingesetztem Elektrodenleitungsstecker gegenüber umgebenden Medien, wie beispielsweise Blut, gewährleistet.

Erfindungsgemäß wird diese Aufgabe durch eine Kontaktbuchse gelöst, die dem Anschluss einer Elektrodenleitung an ein implantierbares medizinisches Gerät dient und die ein Buchsengehäuse besitzt, welches einen längsgestreckten Aufnahmeraum zum Aufnehmen eines Steckers aufweist, und in dem mindestens ein elektrisch leitendes Kontaktelement angeordnet ist. Das elektrisch leitende Kontaktelement verfügt über mindestens einen Federkontakt, der so gestaltet ist, dass er beim Einführen eines Steckers in den Aufnahmeraum federnd elastisch nach außen ausweicht und dort dadurch eine - in Bezug auf den Aufnahmeraum - nach innen wirkende Feder-Rückstellkraft aufbaut, die für einen sicheren Kontakt zwischen dem Federkontakt und einem Gegenkontakt an einem Stecker sorgt.

Erfindungsgemäß umfasst das Kontaktelement wenigstens eine flache Scheibe mit einer zentralen Öffnung, die das Kontaktelement bildende flache Scheibe besitzt zwei ebene Stirnflächen, eine äußere Umfangsfläche und eine, die zentrale Öffnung begrenzende innere Fläche. Der Federkontakt ist Teil der flachen Scheibe und ragt als Vorsprung der inneren Fläche in die zentrale Öffnung hinein. Ein Teil des Federkontaktes bildet eine Kontaktierfläche, die dazu ausgebildet ist, bei einem in die Kontaktbuchse eingeführten Stecker mit einer Gegenkontaktfläche an dem Stecker eine elektrische Verbindung herzustellen.

Ein offensichtlicher Vorteil eines derartigen Kontaktelementes besteht darin, dass eine flache Scheibe leicht herzustellen ist, da sie praktisch aus einem Blech mit einheitlicher Blechdicke ausgeschnitten werden kann. Darüber hinaus hat sich herausgestellt, dass ein derartiges Kontaktelement aufgrund des nach innen ragenden Vorsprungs an den sicheren elektrischen Kontakt mit einer Gegenkontaktfläche eines Elektrodenleitungssteckers herstellen kann.

Die zentrale Öffnung ist - abgesehen von dem Federkontakt oder den Federkontakten - rund.

Gemäß einiger bevorzugter Ausführungsvarianten wölbt sich ein jeweiliger Federkontakt in die zentrale Öffnung der flachen Scheibe hinein.

Außerdem hat es sich als vorteilhaft erwiesen, wenn die Scheibe neben der zentralen Öffnung wenigstens eine zweite Öffnung aufweist, die zwischen dem Federkontakt und der übrigen flachen Scheibe angeordnet und so ausgebildet ist, dass der Federkontakt ein von der zweiten Öffnung und der die zentrale Öffnung begrenzenden inneren Fläche begrenzter Steg ist. Durch Dimensionierung der zweiten Öffnung und des dadurch entstehenden Steges kann die Federkonstante des Federkontaktes - als die beim Auslenken resultierende Federkraft - optimal eingestellt werden.

In einer Variante der flachen Scheibe gehen die zweite Öffnung und die zentrale Öffnung ineinander über, so dass sie eine gemeinsame Öffnung bilden. Dadurch entsteht eine vorzugsweise tangential zur zentralen Öffnung verlaufende Federzunge, die den Federkontakt bildet. Auch diese Ausführungsvariante ist besonders geeignet, so gestaltet zu werden, dass sich eine zum Herstellen des elektrischen Kontaktes zwischen Kontaktelement und eingesetztem Stecker bestens geeignete Federkraft ergibt.

Vorzugsweise weist die flache Scheibe drei Federkontakte auf, die gleichmäßig um die zentrale Öffnung herum angeordnet sind. Eine derartige Anzahl von Federkontaktelementen hat sich als optimal erwiesen.

Neben der flachen Scheibe weist das Kontaktelement vorzugsweise zwei Deckplättchen auf, zwischen denen die flache Scheibe angeordnet ist. Die beiden Deckplättchen weisen dabei jeweils ebenfalls eine zentrale Öffnung auf, deren Durchmesser demjenigen der zentralen Öffnung der flachen Scheibe entspricht, die jedoch keine Federkontakte besitzen. Die flache Scheibe und die beiden Deckplättchen sind so zueinander angeordnet, dass deren zentrale Öffnungen miteinander fluchten. Die beiden Deckplättchen führen dazu, dass die von der flachen Scheibe gebildeten Federkontakte in längsaxialer Richtung des Buchsengehäuses stabilisiert werden und in erster Linie in radialer Richtung ausfedern.

Sowohl die flache Scheibe als auch die Deckplättchen haben - mit Ausnahme einer vorzugsweise vorgesehenen Kontaktfahne - jeweils eine runde Außenkontur. Dabei sind die Außendurchmesser der flachen Scheibe sowie der Deckplättchen vorzugsweise ebenfalls einander identisch.

Um die Deckplättchen und die flache Scheibe optimal zueinander ausrichten zu können, sind vorzugsweise wenigstens zwei Positionierlöcher vorgesehen, die bei optimaler Ausrichtung der Deckplättchen und der Zwischenlinien angeordneten flachen Scheibe miteinander fluchten.

Darüber hinaus sind die Deckplättchen mit der Scheibe vorzugsweise fest verbunden. Ein derartiger fester Verbund kann beispielsweise durch Punktschweißen geschehen. Hierzu können die Deckplättchen und die flache Scheibe mittels der Positionierlöcher auf zwei Stifte aufgefädelt werden, die die Deckplättchen und die flache Scheibe in Position zueinander halten, um dann Deckplättchen und flache Scheibe miteinander zu verschweißen. Alternativ könnten die Positionierlöcher auch der Aufnahme jeweils eines Nietes zum Verbinden von Deckplättchen und flacher Scheibe dienen.

Geeignete Materialien für die flache Scheibe sind beispielsweise eine Platin-Iridium-Legierung wie PT-IR(80/20) oder (70/30), reines Platin, TiAl6V4, Stahllegierungen wie beispielsweise MP35N (ISO-5832-6, ASTM-F 688-00), 316L (Werkstoffnr. nach EN 10088-1, -2, -3: 1.4404, 1.4435) oder 316LVM (ISO-5832-1, Werkstoffnr. nach EN 10088-1, -2, -3: 1.4441) oder andere implantatsgeeignete metallische Werkstoffe nach ISO-5832 oder ASTM.

Die Kontaktbuchse ist vorzugsweise Teil eines Anschlussstücks eines Herzschrittmachers oder eines Kardioverters/Defibrillators oder einer Kombination aus beiden.

Neben einer vollständigen Kontaktbuchse der beanspruchten Art wird außerdem ein einzelnes Kontaktelement für eine solche Kontaktbuchse beansprucht.

Die Erfindung soll nun anhand eines Ausführungsbeispiels mit Bezug auf die Figuren näher erläutert werden. Von den Figuren zeigen:
- Figur 1:: eine schematische Seitenansicht eines Herzschrittmachers;
- Figur 2:: eine Seitenansicht eines Elektrodenleitungssteckers;
- Figur 3:: einen Längsschnitt durch eine Kontaktbuchse des Herzschrittmacher aus Figur 1;

- Figur 4:: ein Kontaktelement als Bestandteil der Kontaktbuchse aus Figur 3 mit einer zentralen flachen Scheibe und zwei Deckplättchen in einer Explosionsansicht;
- Figur 5:: eine Draufsicht auf eine erste Variante einer flachen Scheibe für das Kontaktelement aus Figur 4;
- Figur 6:: eine Draufsicht auf eine zweite Variante einer flachen Scheibe für das Kontaktelement aus Figur 4;
- Figur 7:: eine Draufsicht auf eine dritte Variante einer flachen Scheibe für das Kontaktelement aus Figur 4;
- Figur 8:: eine vierte Variante einer flachen Scheibe für das Kontaktelement aus Figur 4; und
- Figur 9:: eine Draufsicht auf ein Deckplättchen für das Kontaktelement aus Figur 4.

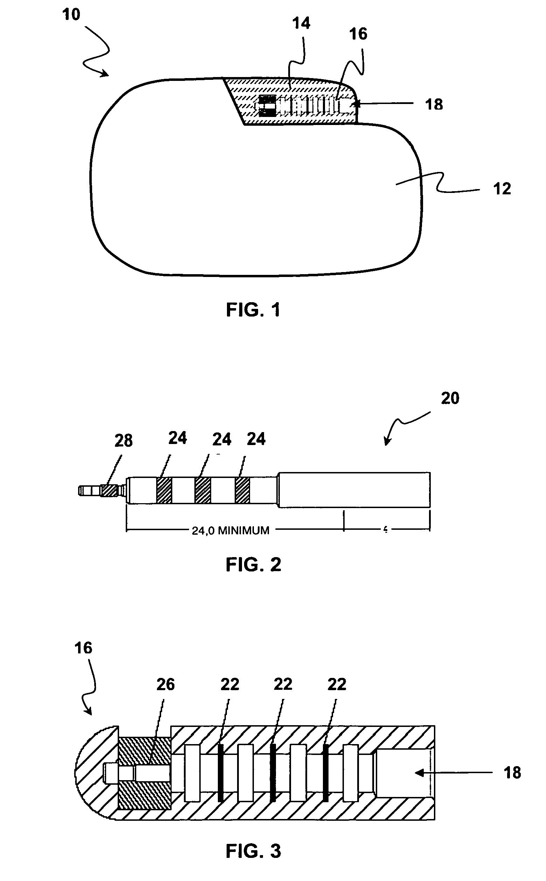

Figur 1 zeigt einen Herzschrittmacher 10 mit einem Metallgehäuse 12, das elektrische und/oder elektronische Komponenten des Herzschrittmachers beherbergt und einem dicht mit dem Metallgehäuse 12 verbundenen Anschlussgehäuse 14. Das Anschlussgehäuse 14 beherbergt eine Kontaktbuchse 16, deren wesentlicher Bestandteil ein Aufnahmeraum 18 zur Aufnahme eines Elektrodenleitungssteckers 20 ist, wie er in Figur 2 abgebildet ist. Figur 3 ist eine vergrößerte Ansicht der Kontaktbuchse 16 und ihres Aufnahmeraums 18 im Längsschnitt.

Die Kontaktbuchse weist drei gleichartige Kontaktelemente 22 auf, die dem Kontaktieren von ringförmigen Gegenkontaktflächen 24 des Elektrodenleitungssteckers 20 dienen.

Darüber hinaus weist die Kontaktbuchse 16 ein viertes Kontaktelement 26 auf, das zur Aufnahme eines Terminal-Schraubkontaktes 28 am äußeren freien Ende des Elektrodenleitungssteckers 20 dient.

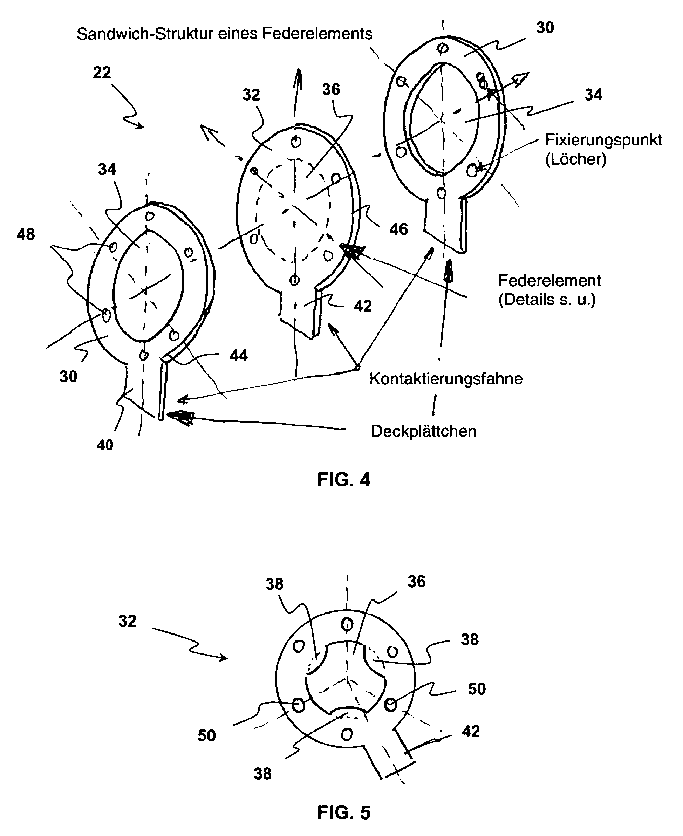

Figur 4 zeigt beispielhaft eines der Kontaktelemente 22 in einer Explosionsdarstellung. Jedes der Kontaktelemente 22 besteht aus zwei Deckplättchen 30 und einer zwischen diesen beiden Deckplättchen 30 angeordneten flachen Scheibe 32.

Sowohl die beiden Deckplättchen 30 als auch die flache Scheibe 32 werden jeweils von einem flachen Blech gebildet, das beispielsweise aus MP35N oder TiAI6V4 besteht.

Sowohl die Deckplättchen 30 als auch die flache Scheibe 32 weisen jeweils eine zentrale Öffnung 34 beziehungsweise 36 auf, die in Bezug auf die flache Scheibe 32 in Figur 3 nur angedeutet dargestellt ist.

Während die zentralen Öffnungen 34 der Deckplättchen 30 kreisrund sind, hat die zentrale Öffnung 36 der flachen Scheibe 32 zwar eine kreisrunde Grundform, weist jedoch zusätzlich jeweils drei Federkontakte 38 auf, die in die zentrale Öffnung 36 der flachen Scheibe 32 hineinragen.

Die äußere Kontur der Deckplättchen 30 und der flachen Scheibe 32 besitzen eine bezüglich der jeweiligen zentralen Öffnung 34 beziehungsweise 36 konzentrische, kreisrunde Grundform, die jeweils an einer Umfangsposition eine Ausbuchtung aufweist, die jeweils eine Kontaktierfahne 40 beziehungsweise 42 bildet. Die durch eine jeweilige Umfangsfläche 44 beziehungsweise 46 gebildeten Umfangskonturen der Deckplättchen 30 und der flachen Scheibe 32 sind einander identisch.

Wie Figur 4 zu entnehmen ist, sind Deckplättchen 30 und die flache Scheibe 32 eines jeweiligen Federkontaktes 22 im fertig montierten Zustand so zueinander ausgerichtet, dass deren zentrale Öffnungen 34 beziehungsweise 36 und deren Kontaktierfahnen 40 beziehungsweise 42 deckungsgleich übereinander liegen.

Um dies zu bewirken, sind sowohl bei den Deckplättchen 30 als auch bei der flachen Scheibe 32 jeweils insgesamt 6 Positionierlöcher 48 beziehungsweise 50 vorgesehen, die bei fertig montiertem Kontaktelement 22 jeweils miteinander fluchten. Die Positionierlöcher 48 beziehungsweise 50 sind auf einem mit der jeweiligen zentralen Öffnung 34 beziehungsweise 36 konzentrisch im Kreis gleichmäßig verteilt.

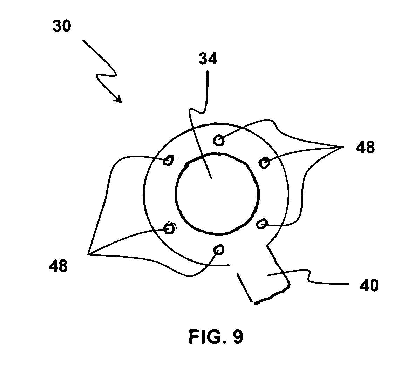

Erfindungsgemäß sind verschiedene Varianten von flachen Scheiben 32 vorgesehen. Diese sind in den Figuren 5 bis 8 abgebildet und unterscheiden sich von den in Figur 9 noch einmal abgebildeten Deckplättchen 30 dadurch, dass die flache Scheibe jeweils drei Federkontakte 38 aufweist.

Bei der in Figur 5 dargestellten Ausführungsvariante sind die drei gleichmäßig über den Umfang der zentralen Öffnung 36 angeordneten Federkontakte 38 sich in die zentrale Öffnung 36 hineinwölbende Vorsprünge. Bei dieser Ausführungsvariante bewirken die Federkontakte 38 eine recht hohe Klemmkraft auf entsprechende Gegenkontaktflächen eines in die Kontaktbuchse 16 eingesetzten Elektrodenleitungssteckers 20.

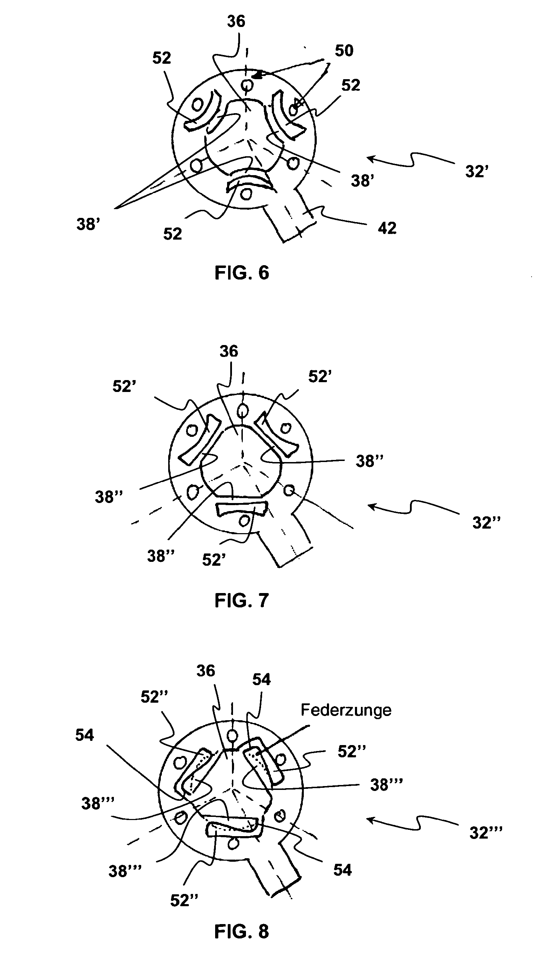

Eine geringere Klemmkraft in Folge einer geringeren Federkraft beim Auslenken der Federkontakte 38' ergibt sich bei der in Figur 6 dargestellten Ausführungsvariante einer flachen Scheibe 32'. Bei dieser sind die Federkontakte 38' von schmalen Stegen gebildet, die auf der Innenseite durch die zentrale Öffnung 36 begrenzt sind und auf ihrer in radialer Richtung nach außen gewandten Seite durch eine jeweilige zweite Öffnung 52. Die jeweilige zweite Öffnung 52 ist kreisbogenförmig in Richtung des Zentrums der zentralen Öffnung 36 gewölbt und so angeordnet, dass der jeweilige einen Federkontakt 38' bildende Steg wenigstens annähernd eine einheitliche Stärke hat.

In Figur 7 ist eine Ausführungsvariante einer flachen Scheibe 32" dargestellt, die der in Figur 6 dargestellten Ausführungsvariante stark ähnelt. Der wesentliche Unterschied besteht darin, dass die Federkontakte 38" bei der Ausführungsvariante gemäß Figur 7 keine in Bezug auf die zentrale Öffnung 36 nach innen gewölbten Stege sind, sondern gerade Stege, die Federkontakte 38" bilden somit Sekanten zur zentralen Öffnung 36. Dadurch, dass die Federkontakte 38" gestreckt sind, ergibt sich im Vergleich zu der Ausführungsvariante gemäß Figur 6 geringere Federkraft, wenn ein Stecker 20 in die Kontaktbuchse 16 eingesetzt wird.

Figur 8 ist schließlich eine Ausführungsvariante einer flachen Scheibe 32''' dargestellt, bei der die zweiten Öffnungen 52" in die zentrale Öffnung 36 übergehen, so dass sich eine einzige Gesamtöffnung ergibt. Hierdurch ergeben sich Federkontakte 38"', die nur an einem Ende mit der übrigen flachen Scheibe 32''' verbunden sind und ansonsten ein freies Ende 54 aufweisen und somit jeweils eine Federzunge bilden. Bei dieser Ausführungsvariante haben die Federkontakte 38 eine noch geringe Federkonstante, als die Federkontakte 38" der Ausführungsvariante gemäß Figur 7.

Ein fertiges Kontaktelement 22 wird jeweils von zwei Deckplättchen 30 (siehe Figuren 4 und 9) und einer flachen Scheibe gemäß einer der Figuren 5 bis 8 gebildet. Die Deckplättchen 30 und die flache Scheibe 32 werden vor dem Einsetzen in die Kontaktbuchse 16 beispielsweise durch Schweißen fest miteinander verbunden. Nach dem Einsetzen in die Kontaktbuchse 16 werden elektrische Verbindungsleitungen an die dann gemeinsame Kontaktierfahne 40 beziehungsweise 42 des Kontaktelementes 22 geschweißt oder gelötet.

## Patentansprüche

1. Kontaktbuchse (16) zum Anschluss einer Elektrodenleitung an ein implantierbares medizinisches Gerät, mit
einem Buchsengehäuse, welches einen längsgestreckten Aufnahmeraum (18) mit einer Längsachse zum Aufnehmen eines Steckers entlang der Längsachse aufweist, und
wenigstens einem in dem Buchsengehäuse angeordneten elektrisch leitenden Kontaktelement (22), welches über wenigstens einen Federkontakt (38) verfügt, der beim Einführen eines Steckers federnd elastisch nach außen ausweicht, wodurch eine nach innen wirkende Feder-Rückstellkraft aufgebaut wird,
**dadurch gekennzeichnet, dass**
das Kontaktelement (22) wenigstens eine flache Scheibe (32) mit einer zentralen Öffnung umfasst, welche über zwei ebene Stirnflächen, sowie eine äußere Umfangsfläche und eine, die zentrale Öffnung begrenzende innere Fläche verfügt, wobei der Federkontakt (38) Teil der flachen Scheibe (32) ist, als Vorsprung der inneren Fläche in die zentrale Öffnung (36) hineinragt und über eine Kontaktierfläche verfügt, die dazu ausgebildet ist, bei in die Kontaktbuchse eingeführtem Stecker mit einer Gegenkontaktfläche des Steckers eine elektrische Verbindung herzustellen.

2. Kontaktbuchse (16) nach Anspruch 1, **dadurch gekennzeichnet, dass** die zentrale Öffnung (36) abgesehen von dem Federkontakt (38) rund ist.

3. Kontaktbuchse (16) nach Anspruch 1 oder 2, **dadurch gekennzeichnet, dass** die Scheibe (32) wenigstens eine nach außen weisende Ausbuchtung (42) der äußeren Umfangsfläche aufweist, die als eine Kontaktierfahne dient.

4. Kontaktbuchse (16) nach einem der Ansprüche 1 bis 3, **dadurch gekennzeichnet, dass** sich der Federkontakt (38, 38') in die zentrale Öffnung hineinwölbt.

5. Kontaktbuchse (16) nach einem der Ansprüche 1 bis 4, **dadurch gekennzeichnet, dass** die Scheibe (32'; 32"; 32''') neben der zentralen Öffnung (36) über wenigstens eine zweite Öffnung (52; 52'; 52") verfügt, die der Nähe der inneren Fläche im Bereich des Vorsprungs so angeordnet und ausgebildet ist, dass der Federkontakt (38', 38", 38''') ein von der zweiten Öffnung und der inneren Fläche begrenzter Steg ist.

6. Kontaktbuchse (16) nach Anspruch 5, **dadurch gekennzeichnet, dass** die zweite Öffnung (52") an einem Längsende zur zentralen Öffnung (36) hin geöffnet ist, so dass sich eine tangential zur zentralen Öffnung verlaufende Federzunge (38''') ergibt, die den Federkontakt bildet.

7. Kontaktbuchse (16) nach einem der Ansprüche 1 bis 4, **dadurch gekennzeichnet, dass** die Scheibe (32; 32'; 32"; 32''') drei Federkontakte aufweist, die in Bezug auf den Mittelpunkt um jeweils 120° zueinander versetzt angeordnet sind.

8. Kontaktbuchse (16) nach einem der Ansprüche 1 bis 7, **dadurch gekennzeichnet, dass** das Kontaktelement (22) die Scheibe (32) und zwei Deckplättchen (30) umfasst, wobei die Scheibe (32) in Buchsenlängsrichtung zwischen den Deckplättchen (30) angeordnet ist und die Deckplättchen (30) ebenfalls jeweils eine zentrale Öffnung (34) aufweisen, die mit der zentralen Öffnung (36) der flachen Scheibe (32) fluchten.

9. Kontaktbuchse (16) nach Anspruch 8, **dadurch gekennzeichnet, dass** jedes Deckplättchen (30) den gleichen Außendurchmesser wie die Scheibe (32) besitzt.

10. Kontaktbuchse (16) nach einem der Ansprüche 6 oder 7, **dadurch gekennzeichnet, dass** sowohl die Deckplättchen (30), als auch die Scheibe (32) jeweils über wenigstens zwei Positionierlöcher (50) verfügen, die miteinander fluchtend angeordnet sind.

11. Kontaktbuchse (16) nach einem der Ansprüche 8 bis 10, **dadurch gekennzeichnet, dass** die Deckplättchen (30) fest mit der Scheibe (32) verbunden sind.

12. Kontaktbuchse (16) nach einem der Ansprüche 1 bis 11, **dadurch gekennzeichnet, dass** die Scheibe (32) zumindest teilweise aus Pt/Ir (80/20); (70/30) oder Pt oder TiAl6V4 oder 316L oder 316LVM oder MP35N besteht.

13. Kontaktbuchse (16) nach einem der Ansprüche 1 bis 12, **dadurch gekennzeichnet, dass** die Kontaktbuchse (16) Teil eines Anschlussstücks (14) eines Herzschrittmachers und/oder eines Kardioverters/Defibrillators (10) ist.

14. Kontaktelement (22) für eine Kontaktbuchse gemäß einem der Ansprüche 1 bis 13.
